# EUROPEAN PATENT APPLICATION

(11) **EP 4 006 068 A1**
(43) Date of publication of application: **01.06.2022**
(21) Application number: 20853675.5
(22) Date of filing: 21.08.2020
(51) Int. Cl.: C08F 220/38, A61L 29/06, A61L 29/08, A61L 29/12, A61L 29/14, A61L 31/06, A61L 31/10, A61L 31/12, A61L 31/14

(54) **MEDICAL INSTRUMENT AND MANUFACTURING METHOD THEREFOR**

(30) Priority: 21.08.2019 JP 2019151586
(71) Applicant: TERUMO Kabushiki Kaisha, Tokyo 151-0072 (JP)
(72) Inventor: KURAMOTO, Masanori, Ashigarakami-gun, Kanagawa 259-0151 (JP)
(74) Representative: Casalonga
(86) International application number: PCT/JP2020/031643
(87) International publication number: WO 2021/033767

(57) **Abstract**

The invention provides a medical device that exhibits an excellent lubricating property. The medical device according to the invention includes: a substrate layer; and a surface lubricious layer formed on at least a part of the substrate layer and containing a polymer containing a structural unit derived from acrylamide, and a hydrophilic copolymer containing a structural unit derived from a polymerizable monomer (A) having a sulfobetaine structure, a structural unit derived from a polymerizable monomer (B) having at least one group selected from a group consisting of a sulfonic acid group (-SO₃H), a sulfuric acid group (-OSO₃H), a sulfurous acid group (-OSO₂H), and salt groups thereof, and a structural unit derived from a polymerizable monomer (C) having a photoreactive group.

## Description

### Technical Field

The present invention relates to a medical device and a method for manufacturing the same. In particular, the invention relates to a medical device including a surface lubricious layer exhibiting an excellent lubricating property and a method for manufacturing the same.

### Background Art

In recent years, a catheter has a reduced outer diameter to improve an insertion property thereof to a peripheral portion of a blood vessel, and is thereby used for diagnosis and treatment of various lesion sites. Therefore, in the diagnosis or the treatment using the catheter, a clearance between the catheter and an inner surface of a lumen in a living body is extremely small, which may result in high frictional resistance on a surface of the catheter. Therefore, the catheter is required to include a coating that imparts a lubricating property and durability (lubrication retaining property) to the surface of the catheter.

For example, WO 2018/038063 (corresponding to US 2019/0185776 A1) discloses that a hydrophilic copolymer is used for a surface lubricious layer, the hydrophilic copolymer containing a structural unit derived from a polymerizable monomer (A) having a sulfobetaine structure, a structural unit derived from a polymerizable monomer (B) having a group such as a sulfonic acid group, and a structural unit derived from a polymerizable monomer (C) having a photoreactive group.

### Summary of Invention

The surface lubricious layer disclosed in WO 2018/038063 (corresponding to US 2019/0185776 A1) certainly exhibits an excellent lubricating property and excellent durability (lubrication retaining property). On the other hand, a medical technique for approaching a more flexible medical device to a narrower lesion site in a living body becomes widespread, and in recent years, a demand for operability for making the medical device reach the lesion site increases. Therefore, a technique for further improving the lubricating property in order to operate the medical device satisfactorily even in a narrower lesion site is demanded.

Therefore, an object of the invention is to provide a means for improving the lubricating property.

The present inventor has made diligent studies to solve the above problem. As a result, the present inventor has found that the above problem can be solved by providing, on a substrate layer, a surface lubricious layer containing a hydrophilic copolymer containing specific structural units and a polymer containing a structural unit derived from acrylamide, and has thus completed the invention.

That is, the above object can be achieved by a medical device including: a substrate layer; and a surface lubricious layer formed on at least a part of the substrate layer and containing a polymer containing a structural unit derived from acrylamide, and a hydrophilic copolymer containing a structural unit derived from a polymerizable monomer (A) having a sulfobetaine structure, a structural unit derived from a polymerizable monomer (B) having at least one group selected from a group consisting of a sulfonic acid group (-SO₃H), a sulfuric acid group (-OSO₃H), a sulfurous acid group (-OSO₂H), and salt groups thereof, and a structural unit derived from a polymerizable monomer (C) having a photoreactive group.

### Brief Description of Drawings

[Fig. 1] Fig. 1 is a partial cross-sectional view schematically showing a surface lamination structure of a medical device according to an exemplary embodiment of the invention.
[Fig. 2] Fig. 2 is a partial cross-sectional view schematically showing a configuration example having a different surface lamination structure as an application example of the embodiment in Fig. 1.
[Fig. 3] Fig. 3 is a schematic view showing a lubricating property and durability test device (friction meter) used in Examples and Comparative Examples.
[Fig. 4] Fig. 4 is a graph showing lubricating property and durability test results in Example 1 and Comparative Example 1.

### Description of Embodiments

Hereinafter, an embodiment of the invention will be described in detail. Note that the invention is not limited to the following embodiment. In the present description, "X to Y" indicating a range includes X and Y, and means "X or more and Y or less". In the present description, "X and/or Y" means to include at least one of X and Y, and includes "X alone", "Y alone", and "a combination of X and Y". Unless otherwise specified, operations, measurements of physical properties, and the like are performed under conditions of room temperature (20°C to 25°C) and a relative humidity of 40% to 60% RH.

In the present description, the term "(meth)acrylic" includes both acrylic and methacrylic. Therefore, for example, the term "(meth)acrylic acid" includes both acrylic acid and methacrylic acid. Similarly, the term "(meth)acryloyl" includes both acryloyl and methacryloyl. Therefore, for example, the term "(meth)acryloyl group" includes both an acryloyl group and a methacryloyl group.

In the present description, unless otherwise specified, the term "substituted" refers to being substituted with a C1 to C30 alkyl group, a C2 to C30 alkenyl group, a C2 to C30 alkynyl group, a C1 to C30 alkoxy group, an alkoxycarbonyl group (-COOR, R represents a C1 to C30 alkyl group), a halogen atom (F, Cl, Br, or I atom), a C6 to C30 aryl group, a C6 to C30 aryloxy group, an amino group, a C1 to C30 alkylamino group, a cyano group, a nitro group, a thiol group, a C1 to C30 alkylthio group, or a hydroxy group. Note that, when a group is substituted, a substitution in which a structure after substitution falls under a definition before the substitution is excluded. For example, when a substituent is an alkyl group, this alkyl group as a substituent is not further substituted with another alkyl group.

In the present description, a "polymerizable monomer (A) having a sulfobetaine structure" is also simply referred to as a "polymerizable monomer (A)" or a "polymerizable monomer (A) according to the invention". Similarly, a "structural unit derived from a polymerizable monomer (A) having a sulfobetaine structure" is also simply referred to as a "structural unit (A)" or a "structural unit (A) according to the invention".

In the present description, a "polymerizable monomer (B) having at least one group selected from the group consisting of a sulfonic acid group (-SO₃H), a sulfuric acid group (-OSO₃H), a sulfurous acid group (-OSO₂H), and salt groups thereof" is also simply referred to as a "polymerizable monomer (B)" or a "polymerizable monomer (B) according to the invention". Similarly, a "structural unit derived from a polymerizable monomer (B) having at least one group selected from the group consisting of a sulfonic acid group (-SO₃H), a sulfuric acid group (-OSO₃H), a sulfurous acid group (-OSO₂H), and salt groups thereof" is also simply referred to as a "structural unit (B)" or a "structural unit (B) according to the invention".

In the present description, a "polymerizable monomer (C) having a photoreactive group" is also simply referred to as a "polymerizable monomer (C)" or a "polymerizable monomer (C) according to the invention". Similarly, a "structural unit derived from a polymerizable monomer (C) having a photoreactive group" is also simply referred to as a "structural unit (C)" or a "structural unit (C) according to the invention".

In the present description, a "hydrophilic copolymer containing a structural unit (A), a structural unit (B), and a structural unit (C)" is also simply referred to as a "hydrophilic copolymer" or a "hydrophilic copolymer according to the invention".

In the present description, a "polymerizable monomer" is also simply referred to as a "monomer". In the present description, a "structural unit derived from acrylamide" is also simply referred to as an "acrylamide structural unit". A "polymer containing a structural unit derived from acrylamide" is also simply referred to as an "acrylamide-based polymer".

In the present description, when a structural unit is said to be "derived" from a monomer, it means that the structural unit is a divalent structural unit generated by a polymerizable unsaturated double bond (C=C) present in the monomer corresponding to the structural unit becoming a single bond (-C-C-).

According to the invention, a medical device is provided, the medical device including: a substrate layer; and a surface lubricious layer formed on at least a part of the substrate layer and containing a polymer containing a structural unit derived from acrylamide, and a hydrophilic copolymer containing a structural unit derived from a polymerizable monomer (A) having a sulfobetaine structure, a structural unit derived from a polymerizable monomer (B) having at least one group selected from the group consisting of a sulfonic acid group (-SO₃H), a sulfuric acid group (-OSO₃H), a sulfurous acid group (-OSO₂H), and salt groups thereof, and a structural unit derived from a polymerizable monomer (C) having a photoreactive group. The medical device having the above configuration can exhibit an excellent lubricating property.

In recent years, miniaturization and diameter reduction of medical devices are advanced, and a medical technique for approaching a more flexible medical device to a narrower lesion site in a living body becomes widespread. A device that can maintain good operability even in a site where a clearance between the medical device and an inner surface of a lumen in the living body is small is demanded. The present inventor has made diligent studies to meet such a demand. As a result, the present inventor has found that a high lubricating property can be exhibited even under a high load condition (that is, even in a site where the clearance between the medical device and the inner surface of the lumen in the living body is small) by providing, on the substrate layer, the above surface lubricious layer containing the hydrophilic copolymer and the polymer containing a structural unit derived from acrylamide. A mechanism by which such an effect is produced is unclear, but the following mechanism is estimated. Note that the following mechanism is estimation, and the invention is not limited to the following estimation. Specifically, the hydrophilic copolymer contained in the surface lubricious layer exhibits a lubricating property when wet (for example, when in contact with an aqueous liquid such as a body fluid or physiological saline). According to the invention, the surface lubricious layer contains the polymer containing a structural unit derived from acrylamide (acrylamide-based polymer) in addition to the hydrophilic copolymer. The acrylamide-based polymer has a water retention effect (acts as a water-retaining material). In such a surface lubricious layer, in addition to a water absorption effect of the acrylamide-based polymer, a crosslink density is moderately low due to the presence of the acrylamide-based polymer. Therefore, the aqueous liquid easily enters the surface lubricious layer (the hydrophilic copolymer easily exhibits a lubricating property (surface gel hydration lubrication) when in contact with the aqueous liquid). Under a high load condition, the surface lubricious layer according to the invention can maintain a sufficient hydrated layer on a surface of the medical device due to the aqueous liquid retained on the surface lubricious layer. Therefore, it is considered that the hydrophilic copolymer can exhibit a sufficient lubricating property even under a high load condition.

The hydrophilic copolymer contained in the surface lubricious layer has a photoreactive group. When the surface lubricious layer is irradiated with active energy rays, the photoreactive group generates reactive species, and the hydrophilic copolymer in the surface lubricious layer reacts with a material constituting a layer adjacent to the surface lubricious layer (for example, the substrate layer) to form a covalent bond between the surface lubricious layer and the layer adjacent to the surface lubricious layer. In addition, the hydrophilic copolymer in the surface lubricious layer reacts with the acrylamide-based polymer in the surface lubricious layer, so that the acrylamide-based polymer is firmly immobilized in the surface lubricious layer. Accordingly, the medical device according to the invention can maintain an initial lubricating property for a longer period of time and have further improved durability (lubrication retaining property).

Therefore, the medical device according to the invention can exhibit an excellent lubricating property even under a condition where the clearance between the medical device and the inner surface of the lumen in the living body is small (high load condition). In addition, the medical device according to the invention can exhibit excellent durability (lubrication retaining property).

Hereinafter, a preferred embodiment of the medical device according to the invention will be described with reference to the attached drawings.

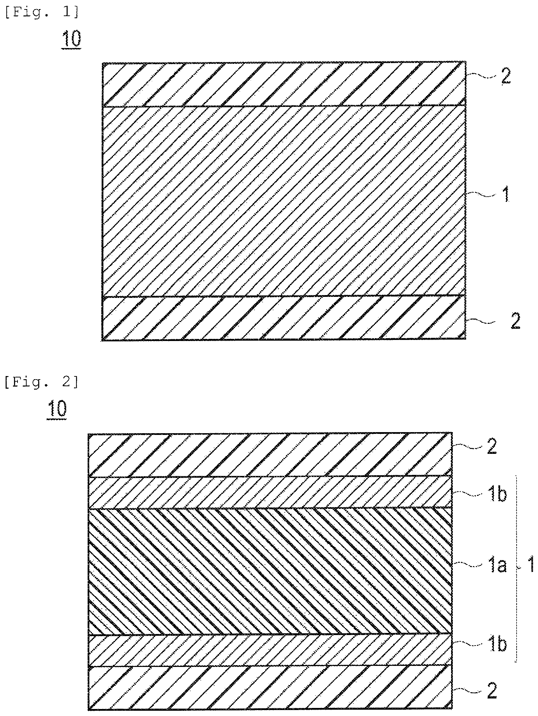

Fig. 1 is a partial cross-sectional view schematically showing a surface lamination structure of a medical device according to an exemplary embodiment of the invention (hereinafter, also simply referred to as a "medical device"). Fig. 2 is a partial cross-sectional view schematically showing a configuration example having a different surface lamination structure as an application example in the present embodiment. Note that in Figs. 1 and 2, 1 represents a substrate layer, 1a represents a substrate layer core portion, 1b represents a substrate surface layer, 2 represents a surface lubricious layer, and 10 represents a medical device.

As shown in Figs. 1 and 2, the medical device 10 according to the present embodiment includes: the substrate layer 1; and the surface lubricious layer 2 containing the hydrophilic copolymer and the polymer containing a structural unit derived from acrylamide and immobilized (disposed) so as to cover at least a part of a surface of the substrate layer 1 (in the drawing, an example of being immobilized (disposed) on the whole surface (entire surface) of the substrate layer 1 in the drawing is shown). The surface lubricious layer 2 is bonded to the substrate layer 1 via the photoreactive group of the hydrophilic copolymer.

Hereinafter, each configuration of the medical device according to the present embodiment will be described.

The invention relates to a medical device including: a substrate layer; and a surface lubricious layer formed on at least a part of the substrate layer and containing a polymer containing a structural unit derived from acrylamide, and a hydrophilic copolymer containing a structural unit derived from a polymerizable monomer (A) having a sulfobetaine structure, a structural unit derived from a polymerizable monomer (B) having at least one group selected from the group consisting of a sulfonic acid group (-SO₃H), a sulfuric acid group (-OSO₃H), a sulfurous acid group (-OSO₂H), and salt groups thereof, and a structural unit derived from a polymerizable monomer (C) having a photoreactive group.

### [Substrate Layer (Substrate)]

The substrate layer used in the invention may be constituted by any material as long as the material can react with the photoreactive group contained in the hydrophilic copolymer, which will be described later, to form a chemical bond. Specifically, examples of the material constituting (forming) the substrate layer 1 include a metal material, a polymer material, and ceramics. Here, as shown in Fig. 1, the substrate layer 1 may be entirely (wholly) constituted (formed) by any one of the above materials, or, as shown in Fig. 2, the substrate layer 1 may have a configuration in which a surface of the substrate layer core portion 1a constituted (formed) by any one of the above materials is covered (coated) with any other of the above materials by an appropriate method to constitute (form) the substrate surface layer 1b. Examples of the latter case include a configuration in which a metal material is covered (coated) by an appropriate method (a known method in the related art such as plating, metal deposition, and sputtering) on the surface of the substrate layer core portion 1a formed by a resin material or the like to form the substrate surface layer 1b, and a configuration in which on the surface of the substrate layer core portion 1a formed by a hard reinforcing material such as a metal material or a ceramic material, a polymer material that is more flexible than the reinforcing material such as a metal material is covered (coated) by an appropriate method (a known method in the related art such as immersion (dipping), spraying, coating, and printing), or the reinforcing material of the substrate layer core portion 1a and the polymer material of the substrate surface layer 1b are composited (an appropriate reaction treatment), so as to form the substrate surface layer 1b. Therefore, the substrate layer core portion 1a may be a multilayer structure in which different materials are laminated in multiple layers, a structure (composite) in which members made of different materials for each part of the medical device are connected to each other, or the like. Another middle layer (not shown) may be formed between the substrate layer core portion 1a and the substrate surface layer 1b. The substrate surface layer 1b may also be a multilayer structure in which different materials are laminated in multiple layers, a structure (composite) in which members made of different materials for each part of the medical device are connected to each other, or the like.

Among the materials constituting (forming) the above substrate layer 1, the metal material is not particularly limited, and metal materials commonly used in medical devices such as a catheter, a stent and a guide wire are used. Specific examples thereof include various stainless steels (SUS) such as SUS304, SUS316, SUS316L, SUS420J2, and SUS630, gold, platinum, silver, copper, nickel, cobalt, titanium, iron, aluminum, tin, and various alloys such as a nickel-titanium (Ni-Ti) alloy, a nickel-cobalt (Ni-Co) alloy, a cobalt-chromium (Co-Cr) alloy, and a zinc-tungsten (Zn-W) alloy. These metal materials may be used alone or in combination of two or more types thereof. The most suitable metal material as a substrate layer for a catheter, a stent, a guide wire, or the like, which is the intended use, may be appropriately selected for the above metal materials.

Among the materials constituting (forming) the above substrate layer 1, the polymer material is not particularly limited, and polymer materials commonly used in medical devices such as a catheter, a stent and a guide wire are used. Specific examples thereof include polyamide resins, polyethylenes such as a linear low density polyethylene (LLDPE), a low density polyethylene (LDPE), a high density polyethylene (HDPE), and a modified polyethylene, polyolefin resins such as polypropylene, polyester resins such as polyethylene terephthalate, polystyrene resins such as polystyrene, cyclic polyolefin resins, modified polyolefin resins, epoxy resins, urethane resins, diallyl phthalate resins (allyl resin), polycarbonate resins, fluororesin, amino resins (a urea resin, a melamine resin, and a benzoguanamine resin), acrylic resins, polyacetal resins, vinyl acetate resins, phenol resins, vinyl chloride resins, silicone resins (silicon resins), polyether resins such as polyetheretherketone (PEEK), and polyimide resins. These polymer materials may be used alone or in combination of two or more types thereof. The most suitable polymer material as a substrate layer for a catheter, a stent, a guide wire, or the like, which is the intended use, may be appropriately selected for the above polymer materials.

A shape of the above substrate layer is not particularly limited, and is appropriately selected as a sheet shape, a linear (wire) shape, a tubular shape, and the like depending on the form of the substrate layer to be used.

### [Surface Lubricious Layer]

The surface lubricious layer in the invention is formed on at least a part of the substrate layer and contains (i) the polymer containing a structural unit derived from acrylamide, and (ii) the hydrophilic copolymer containing the structural unit derived from the polymerizable monomer (A) having a sulfobetaine structure, the structural unit derived from the polymerizable monomer (B) having at least one group selected from the group consisting of a sulfonic acid group (-SO₃H), a sulfuric acid group (-OSO₃H), a sulfurous acid group (-OSO₂H), and salt groups thereof, and the structural unit derived from the polymerizable monomer (C) having a photoreactive group.

Here, the surface lubricious layer is not necessarily formed on the entire surface of the substrate layer. The surface lubricious layer may be formed on a surface portion (a part) of the substrate layer to be in contact with the body fluid.

The surface lubricious layer contains (i) the polymer containing a structural unit derived from acrylamide (acrylamide-based polymer) and (ii) the hydrophilic copolymer. Of these, (ii) the hydrophilic copolymer exhibits a lubricating property when wet (for example, when in contact with an aqueous liquid such as a body fluid or physiological saline). (i) The acrylamide-based polymer acts to retain the aqueous liquid. The presence of the acrylamide-based polymer moderately reduces the crosslink density of the surface lubricious layer. Therefore, the aqueous liquid easily enters the surface lubricious layer, and the hydrophilic copolymer easily exhibits a lubricating property (gel hydration lubrication). In addition, under a high load condition, the surface lubricious layer forms a hydrated layer between the inner surface of the lumen in the living body and the medical device due to the aqueous liquid retained on the surface lubricious layer. Therefore, it is considered that the hydrophilic copolymer can exhibit a lubricating property by being in contact with a sufficient amount of the aqueous liquid even under a high load condition. Note that the above mechanism is estimation, and the invention is not limited to the above estimation.

In the invention, another layer may be provided between the surface lubricious layer and the substrate layer as long as another layer does not influence functions and effects of the invention, and preferably, the surface lubricious layer is directly disposed above the substrate layer. In addition, another layer may be provided on the surface lubricious layer as long as the functions and effects of the invention are not influenced, and it is preferable that another layer is not disposed on the surface lubricious layer (the surface lubricious layer is an outermost layer). According to this embodiment, the effect (lubricating property) of the invention can be effectively exhibited.

A thickness of the surface lubricious layer is not particularly limited. From the viewpoints of the lubricating property, the durability (lubrication retaining property), the adhesiveness to the adjacent layer (for example, the substrate layer), and the like, the thickness (dry film thickness) of the surface lubricious layer is preferably 0.01 µm to 100 µm, and more preferably 0.1 µm to 50 µm.

Hereinafter, compositions (the polymer containing a structural unit derived from acrylamide, the hydrophilic copolymer, and the like) contained in the surface lubricious layer according to the invention will be described.

### (Polymer Containing Structural Unit Derived from Acrylamide)

The surface lubricious layer contains the polymer containing a structural unit derived from acrylamide (acrylamide structural unit), i.e., the following formula (acrylamide-based polymer).

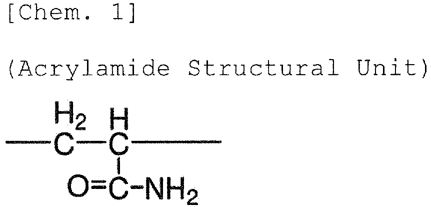

The polymer containing a structural unit derived from acrylamide may contain a structural unit derived from another monomer, in addition to the above acrylamide structural unit. Here, another monomer that may be contained when the acrylamide-based polymer contains the structural unit derived from another monomer is not particularly limited as long as another monomer does not inhibit the water retention effect. Specific examples thereof include N,N-dimethylacrylamide, N-isopropylacrylamide, vinylpyrrolidone, acrylic acid, and acrylates (for example, sodium salt and potassium salt). Here, the structural unit derived from the above another monomer may be one type alone or a combination of two or more types. That is, the structural unit derived from another monomer may be constituted by only one type of structural unit, or may be constituted by two or more types of structural units. Note that a plurality of structural units derived from another monomer may be present in a block shape or in a random shape. In this embodiment, a content of the structural unit derived from another monomer is preferably less than 10 mol%, more preferably less than 5 mol%, and still more preferably less than 1 mol% (lower limit: 0 mol%), with respect to 100 mol%, which is the total of the structural units derived from all the monomers. Note that the mol% is substantially equivalent to a ratio of a charge amount (mol) of another monomer with respect to the total charge amount (mol) of all the monomers in the production of the polymer. Preferably, the polymer containing a structural unit derived from acrylamide according to the invention (acrylamide-based polymer) is preferably constituted by only acrylamide. That is, in a preferred embodiment of the invention, the polymer containing a structural unit derived from acrylamide is polyacrylamide.

A molecular weight of the polymer containing a structural unit derived from acrylamide is not particularly limited. For example, a number average molecular weight (Mn) of the polymer containing a structural unit derived from acrylamide is preferably 500 to 50,000,000, more preferably 1,000 to 25,000,000, still more preferably 10,000 to 10,000,000, particularly preferably 10,000 to 8,000,000, and most preferably 20,000 to 3,000,000. The acrylamide-based polymer in such a range can exhibit a sufficient water retention effect. In the invention, the "number average molecular weight" shall be a value measured by gel permeation chromatography (GPC) using polyethylene glycol as a standard substance.

As the polymer containing a structural unit derived from acrylamide, either a synthetic product or a commercially available product may be used. The commercially available product is available from Sigma-Aldrich Co. LLC. or the like.

### (Hydrophilic Copolymer)

The surface lubricious layer contains the hydrophilic copolymer in addition to the polymer containing a structural unit derived from acrylamide. The hydrophilic copolymer contains the structural unit derived from the polymerizable monomer (A) having a sulfobetaine structure, the structural unit derived from the polymerizable monomer (B) having at least one group selected from the group consisting of a sulfonic acid group (-SO₃H), a sulfuric acid group (-OSO₃H), a sulfurous acid group (-OSO₂H), and salt groups thereof, and the structural unit derived from the polymerizable monomer (C) having a photoreactive group.

In the surface lubricious layer, an abundance ratio (mixing ratio) of the hydrophilic copolymer with respect to the polymer containing a structural unit derived from acrylamide is not particularly limited. In the surface lubricious layer, for the abundance ratio (mixing ratio) of the hydrophilic copolymer with respect to the polymer containing a structural unit derived from acrylamide, the hydrophilic copolymer is preferably 1 part by weight or more, more preferably 1.5 parts by weight or more, still more preferably 2 parts by weight or more, particularly preferably 3 parts by weight or more, and most preferably 4 parts by weight or more, with respect to 1 part by weight of the polymer containing a structural unit derived from acrylamide. In the surface lubricious layer, for the abundance ratio (mixing ratio) of the hydrophilic copolymer with respect to the polymer containing a structural unit derived from acrylamide, the hydrophilic copolymer is preferably 500 parts by weight or less, more preferably 450 parts by weight or less, still more preferably 250 parts by weight or less, particularly preferably 50 parts by weight or less, and most preferably 10 parts by weight or less, with respect to 1 part by weight of the polymer containing a structural unit derived from acrylamide. That is, in a preferred embodiment of the invention, the hydrophilic copolymer is contained in the surface lubricious layer at a ratio of 1 part by weight or more and 500 parts by weight or less (more preferably 1.5 parts by weight to 450 parts by weight, still more preferably 2 parts by weight to 250 parts by weight, particularly preferably 3 parts by weight to 50 parts by weight, and most preferably 4 parts by weight to 10 parts by weight) with respect to 1 part by weight of the polymer containing a structural unit derived from acrylamide. With such an abundance ratio (mixing ratio), the water retention effect of the polymer containing a structural unit derived from acrylamide and the lubricating property of the hydrophilic copolymer can be exhibited in a good balance. Note that when the surface lubricious layer contains two or more types of polymers containing a structural unit derived from acrylamide, the above "1 part by weight" means that a total amount of these polymers containing a structural unit derived from acrylamide is 1 part by weight. Similarly, when the surface lubricious layer contains two or more types of hydrophilic copolymers, the above amount (part by weight) of the hydrophilic copolymer means a total amount of these hydrophilic copolymers. The above abundance ratio (mixing ratio) is substantially equal to a ratio of a total charge amount (weight) of the hydrophilic copolymer with respect to a total charge amount (weight) of the polymer containing a structural unit derived from acrylamide during formation of the surface lubricious layer.

Here, the presence and the ratio (composition) of the structural unit derived from each polymerizable monomer in the hydrophilic copolymer and the presence of the polymer containing a structural unit derived from acrylamide in the surface lubricious layer can be confirmed by, for example, analyzing peak intensity of a group contained in each structural unit using a known method such as IR, NMR, and pyrolysis GC/MS. In the present description, the presence and the ratio of the structural unit derived from each polymerizable monomer in the hydrophilic copolymer in the surface lubricious layer are measured according to the following method.

### (Method for Detecting and Measuring Presence and Ratio of Structural Unit Derived from Each Polymerizable Monomer in Hydrophilic Copolymer in Surface Lubricious Layer)

With the surface of the medical device swollen with heavy water or the like, precision diagonal cutting is performed on the medical device to prepare an inclined cross section of the medical device. From the cross section, a surface lubricious layer portion located on the surface of the substrate of the medical device is cut, and a material of the surface lubricious layer portion is collected. Next, the material of the surface lubricious layer portion is filled into a sample tube for solid NMR without any gap to prepare a sample and NMR measurement is performed. Here, peaks specific to a site (for example, a sulfobetaine structure) specific to the structural unit (A), a site (for example, a salt of a sulfonic acid group) specific to the structural unit (B), and a site (for example, a benzophenone group) specific to the structural unit (C) are to be confirmed, and when these peaks are confirmed, it is determined that the corresponding structural units are present in the sample. A concentration of the site (for example, the sulfobetaine structure) specific to the structural unit (A) (concentration (a)), a concentration of the site (for example, the salt of the sulfonic acid group) specific to the structural unit (B) (concentration (b)), and a concentration of the site (for example, the benzophenone group) specific to the structural unit (C) (concentration (c)) are measured. A ratio of each of the concentrations (a), (b), and (c) is regarded as an abundance ratio of the structural unit derived from each polymerizable monomer in the hydrophilic copolymer. Note that an analyzer and measurement conditions used in the above measurement are as follows.

Analyzer: NM080006, manufactured by JEOL Ltd.
Measurement conditions: heavy water or a mixed liquid of heavy water and a heavy solvent of a lower alcohol.

In addition, the presence of the polymer containing a structural unit derived from acrylamide in the surface lubricious layer can also be confirmed in the same manner as described above (Method for Detecting and Measuring Presence and Ratio of Structural Unit Derived from Each Polymerizable Monomer in Hydrophilic Copolymer in Surface Lubricious Layer). That is, in the above method, a peak specific to a site specific to the polymer containing a structural unit derived from acrylamide (for example, in ¹H-NMR, a proton (hydrogen atom) of an amide group, or in ¹³C-NMR, a carbon atom adjacent to an amide group) is to be confirmed, and when these peaks can be confirmed, it is determined that the polymer containing a structural unit derived from acrylamide is present in the sample.

The abundance ratio (mixing ratio) of the hydrophilic copolymer with respect to the polymer containing a structural unit derived from acrylamide in the surface lubricious layer can also be measured using the same known method as described above. In the present description, the abundance ratio (mixing ratio) of the hydrophilic copolymer with respect to the polymer containing a structural unit derived from acrylamide in the surface lubricious layer is measured according to the following method.

### (Method for Measuring Abundance Ratio (Mixing Ratio) of Hydrophilic Copolymer with respect to Polymer Containing Structural Unit Derived from Acrylamide in Surface Lubricious Layer)

With the surface of the medical device swollen with heavy water or the like, precision diagonal cutting is performed on the medical device to prepare an inclined cross section of the medical device. From the cross section, a surface lubricious layer portion located near the surface of the medical device is cut, and a material of the surface lubricious layer portion is collected. Next, the material of the surface lubricious layer portion is filled into a sample tube for solid NMR without any gap to prepare a sample and NMR measurement is performed. Here, a concentration (concentration (c)) of a site (for example, a benzophenone group) specific to the structural unit (C) is measured. A concentration (concentration (c')) of the copolymer is calculated based on this concentration (c) and the composition of the hydrophilic copolymer. This concentration (c') is regarded as an amount of the hydrophilic copolymer in the surface lubricious layer. Separately, in the same manner, a concentration of a site specific to the polymer containing a structural unit derived from acrylamide (for example, in ¹H-NMR, a proton (hydrogen atom) of an amide group, or in ¹³C-NMR, a carbon atom adjacent to an amide group) (concentration (h)) is measured. This concentration (h) is regarded as an amount of the polymer containing a structural unit derived from acrylamide in the surface lubricious layer. A value obtained by dividing the concentration (c') by the concentration (h) (concentration (c')/concentration (h)) is the abundance ratio (mixing ratio) of the hydrophilic copolymer with respect to the polymer containing a structural unit derived from acrylamide in the surface lubricious layer. Note that an analyzer and measurement conditions used in the above measurement are as follows.

Analyzer: NM080006, manufactured by JEOL Ltd.
Measurement conditions: heavy water or a mixed liquid of heavy water and a heavy solvent of a lower alcohol.

Hereinafter, each polymerizable monomer constituting the hydrophilic copolymer contained in the surface lubricious layer according to the invention will be described.

### (Polymerizable Monomer (A))

The hydrophilic copolymer contains the structural unit derived from the polymerizable monomer (A) having a sulfobetaine structure (structural unit (A)). Here, the structural unit (A) constituting the hydrophilic copolymer may be one type alone or a combination of two or more types. That is, the structural unit (A) may be constituted by only one type of structural unit (A), or may be constituted by two or more types of structural units (A). Note that a plurality of structural units (A) may be present in a block shape or in a random shape.

The polymerizable monomer (A) (monomer A) is a polymerizable monomer having a sulfobetaine structure. The sulfobetaine structure included in the structural unit derived from the monomer A is excellent in effect of imparting the lubricating property. Therefore, the hydrophilic copolymer containing the structural unit derived from the monomer A is considered to be excellent in lubricating property. A homopolymer of the monomer A is soluble in an aqueous NaCl solution, but is insoluble or difficult to dissolve in water or a lower alcohol. Therefore, it is suggested that the sulfobetaine structure may have a strong electrostatic interaction. Therefore, a strong cohesive force acts inside the surface lubricious layer containing the hydrophilic copolymer according to the invention. Accordingly, the surface lubricious layer is considered to have high strength (excellent in durability). Note that the above is estimation, and the invention is not limited to the above estimation.

Here, the "sulfobetaine structure" refers to a structure in which a positive charge and a negative charge containing a sulfur element are present in positions not adjacent to each other, a dissociable hydrogen atom is not bonded to an atom having the positive charge, and a sum of the charges is zero.

The monomer A is not particularly limited, and examples thereof include compounds represented by the following general formulas.

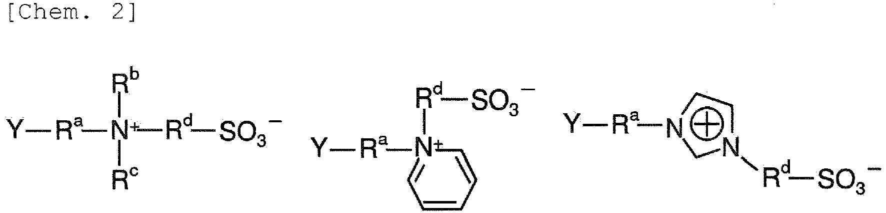

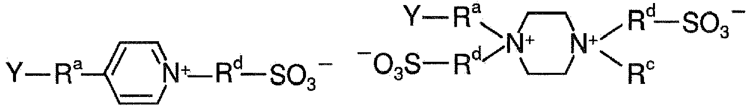

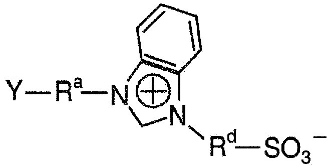

In the above general formulas, R^{a} and R^{d} may each independently represent a substitutable alkylene group having 1 to 30 carbon atoms or a substitutable arylene group having 6 to 30 carbon atoms. R^{b} and R^{c} may each independently represent a substitutable alkyl group having 1 to 30 carbon atoms or a substitutable aryl group having 6 to 30 carbon atoms. Y may represent a group having an ethylenically unsaturated group such as an acryloyl group (CH₂=CH-C(=O)-), a methacryloyl group (CH₂=C(CH₃)-C(=O)-), and a vinyl group (CH₂=CH-). Here, in the above general formulas, the sum of the positive charges and the negative charges is zero.

Examples of the alkylene group having 1 to 30 carbon atoms include a methylene group, an ethylene group, a trimethylene group, a propylene group, an isopropylene group, a butylene group, an isobutylene group, a sec-butylene group, a tert-butylene group, and a pentylene group.

Examples of the arylene group having 6 to 30 carbon atoms include a phenylene group, a naphthylene group, an anthracenylene group, a phenanthrenylene group, a pyrenylene group, a peryleneylene group, a fluorenylene group, and a biphenylene group.

Examples of the alkyl group having 1 to 30 carbon atoms include a methyl group, an ethyl group, a n-propyl group, an isopropyl group, a n-butyl group, an isobutyl group, a sec-butyl group, a tert-butyl group, a n-pentyl group, an iso-amyl group, a tert-pentyl group, a neopentyl group, and a n-hexyl group.

Examples of the aryl group having 6 to 30 carbon atoms include a phenyl group, a biphenyl group, a terphenyl group, a pentarenyl group, an indenyl group, a naphthyl group, an azulenyl group, a heptalenyl group, and a biphenylenyl group.

Among these, from the viewpoint of further improving the lubricating property and the durability (lubrication retaining property), the monomer A is preferably a compound represented by the following formula (1). That is, in a preferred embodiment of the invention, the polymerizable monomer (A) is a compound represented by the following formula (1).

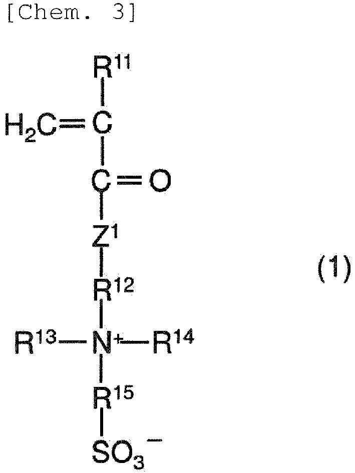

In the above formula (1), R¹¹ represents a hydrogen atom or a methyl group. Z¹ represents an oxygen atom (-O-) or -NH-, and preferably an oxygen atom (-O-).

In the above formula (1), from the viewpoint of further improving the lubricating property and the durability (lubrication retaining property), R¹² and R¹⁵ each independently represent a linear or branched alkylene group having 1 to 20 carbon atoms, preferably a linear or branched alkylene group having 1 to 12 carbon atoms, more preferably a linear or branched alkylene group having 1 to 8 carbon atoms, still more preferably a linear or branched alkylene group having 1 to 6 carbon atoms, even more preferably a linear alkylene group having 1 to 4 carbon atoms (methylene group, ethylene group, trimethylene group, or tetramethylene group), and particularly preferably a linear alkylene group having 1 to 3 carbon atoms (methylene group, ethylene group, or trimethylene group). From the viewpoint of further improving the lubricating property and the durability (lubrication retaining property), as a combination of R¹² and R¹⁵, R¹² preferably represents an ethylene group and R¹⁵ preferably represents a trimethylene group, or R¹² preferably represents a trimethylene group and R¹⁵ preferably represents a tetramethylene group.

In the above formula (1), from the viewpoint of further improving the lubricating property and the durability (lubrication retaining property), R¹³ and R¹⁴ each independently represent a linear or branched alkyl group having 1 to 20 carbon atoms, preferably a linear or branched alkyl group having 1 to 12 carbon atoms, more preferably a linear or branched alkyl group having 1 to 8 carbon atoms, still more preferably a linear or branched alkyl group having 1 to 4 carbon atoms, and particularly preferably a methyl group.

Examples of the compound represented by the above formula (1) include {2-[(meth)acryloyloxy]ethyl}dimethyl-(3-sulfopropyl)ammonium hydroxide, {2-[(meth)acryloyloxy]ethyl}dimethyl-(2-sulfoethyl)ammonium hydroxide, {2-[(meth)acryloyloxy]ethyl}dimethyl-(2-sulfobutyl)ammonium hydroxide, {2-[(meth)acryloyloxy]ethyl}diethyl-(2-sulfoethyl)ammonium hydroxide, {2-[(meth)acryloyloxy]ethyl}diethyl-(3-sulfopropyl)ammonium hydroxide, {2-[(meth)acryloyloxy]ethyl}diethyl-(2-sulfobutyl)ammonium hydroxide, {3-[(meth)acryloyloxy]propyl}dimethyl-(2-sulfoethyl)ammonium hydroxide, {3-[(meth)acryloyloxy]propyl}dimethyl-(3-sulfopropyl)ammonium hydroxide, {3-[(meth)acryloylamino)propyl}dimethyl(3-sulfobutyl)ammonium hydroxide, {3-[(meth)acryloyloxy]propyl}diethyl-(2-sulfoethyl)ammonium hydroxide, {3-[(meth)acryloyloxy]propyl}diethyl-(3-sulfopropyl)ammonium hydroxide, and {3-[(meth)acryloyloxy]propyl}diethyl-(3-sulfobutyl)ammonium hydroxide. Among these, {2-[(meth)acryloyloxy]ethyl}dimethyl-(3-sulfopropyl)ammonium hydroxide and {3-[(meth)acryloyloxy)propyl]dimethyl(3-sulfobutyl)ammonium hydroxide are preferred, {2-[methacryloyloxy]ethyl}dimethyl-(3-sulfopropyl)ammonium hydroxide (MSPB) and [3-(methacryloylamino)propyl]dimethyl(3-sulfobutyl)ammonium hydroxide (MSBB) are more preferred, and {2-[methacryloyloxy]ethyl}dimethyl-(3-sulfopropyl)ammonium hydroxide (MSPB) is still more preferred. The above compounds may be used alone or in combination of two or more types thereof.

As the monomer A, either a synthetic product or a commercially available product may be used. The commercially available product is available from Sigma-Aldrich Co. LLC., FUJIFILM Wako Pure Chemical Corporation, or the like. An exemplary compound may be synthesized with reference to A. Laschewsky, polymers, 6, 1544-1601 (2014), and the like.

The monomer A is not limited to the compounds represented by the above general formulas, and may be a compound having a form in which a positive charge is present at a terminal end.

In the hydrophilic copolymer, when a total of structural units derived from all the monomers is 100 mol%, a content of the structural unit derived from the monomer A is preferably 0.1 mol% to 99 mol%, more preferably 1 mol% to 99 mol%, still more preferably 5 mol% to 99 mol%, and particularly preferably 10 mol% to 99 mol%. Within such a range, a balance between the lubricating property and the solvent solubility is good. Note that when the structural unit (A) is constituted by two or more types of structural units (A), a composition of the above structural unit (A) occupies a ratio (molar ratio (mol%)) of all the structural units (A) with respect to the total of the structural units derived from all the monomers (100 mol%). The mol% is substantially equivalent to a ratio of a charge amount (mol) of the monomer A with respect to a total charge amount (mol) of all the monomers in the production of the polymer.

### (Polymerizable Monomer (B))

The hydrophilic copolymer contains the structural unit derived from the polymerizable monomer (B) having at least one group selected from the group consisting of a sulfonic acid group (-SO₃H), a sulfuric acid group (-OSO₃H), a sulfurous acid group (-OSO₂H), and salt groups thereof (structural unit (B)). Here, the structural unit (B) constituting the hydrophilic copolymer may be one type alone or a combination of two or more types. That is, the structural unit (B) may be constituted by only one type of structural unit (B), or may be constituted by two or more types of structural units (B). Note that a plurality of structural units (B) may be present in a block shape or in a random shape.

The polymerizable monomer (B) (monomer B) is a polymerizable monomer having at least one group selected from the group consisting of a sulfonic acid group (-SO₃H), a sulfuric acid group (-OSO₃H), a sulfurous acid group (-OSO₂H), and salt groups thereof. By introducing such a group, anionization occurs in an aqueous solvent, and electrostatic repulsion occurs between the hydrophilic copolymers. As a result, an electrostatic interaction between the sulfobetaine structures and a hydrophobic interaction between the photoreactive groups in the hydrophilic copolymers are reduced. Therefore, the solvent solubility of the copolymer (particularly the solubility in water, a lower alcohol, or a mixed solvent of water and a lower alcohol) is improved. This improving effect is particularly remarkable when the photoreactive group of the monomer C is a benzophenone group. Since the benzophenone group has a plurality of aromatic rings, the benzophenone groups are likely to associate with each other by a π-π interaction, which makes the polymers containing the benzophenone group to aggregate and insolubilize. Therefore, it is considered that by introducing the structural unit derived from the monomer B, the electrostatic repulsion occurs as described above, and the association between the benzophenone groups is reduced, and thus the solubility or dispersibility of the polymer is rapidly improved. Note that the above mechanism is estimation, and the invention is not limited to the above estimation. Alternatively, even when the monomer C has an ester group, the above improving effect can be obtained satisfactorily. In addition to the above groups, the monomer B preferably has an ethylenically unsaturated group such as a (meth)acryloyl group, a vinyl group, or an allyl group.

Among these, from the viewpoint of further improving the solvent solubility, the monomer (B) is preferably a compound represented by the following formula (2), (3), or (4), and more preferably a compound represented by the following formula (2). That is, in a preferred embodiment of the invention, the polymerizable monomer (B) is a compound represented by the following formula (2), (3), or (4). In a more preferred embodiment of the invention, the polymerizable monomer (B) is a compound represented by the following formula (2).

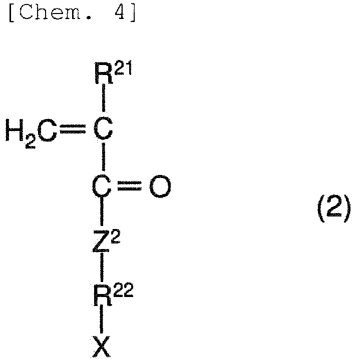

In the above formula (2), R²¹ represents a hydrogen atom or a methyl group. Z² represents an oxygen atom (-O-) or -NH-, and preferably -NH-.

In the above formula (2), from the viewpoint of further improving the solvent solubility, R²² represents a linear or branched alkylene group having 1 to 20 carbon atoms, preferably a linear or branched alkylene group having 1 to 12 carbon atoms, more preferably a linear or branched alkylene group having 1 to 8 carbon atoms, still more preferably a linear or branched alkylene group having 1 to 6 carbon atoms, and particularly preferably a branched alkylene group having 3 to 5 carbon atoms. The branched alkylene group having 3 to 5 carbon atoms is a group represented by -CH(CH₃)-CH₂-, -C(CH₃)₂-CH₂-, -CH(CH₃)-CH(CH₃)-, -C(CH₃)₂-CH₂-CH₂-, -CH(CH₃)-CH(CH₃)-CH₂-, -CH(CH₃)-CH₂-CH(CH₃)-, -CH₂-C(CH₃)₂-CH₂-, -C(CH₃)₂-CH(CH₃)-, or the like (a connection order of the above groups in the above formula (2) is not particularly limited), and among these, a group represented by -C(CH₃)₂-CH₂- is particularly preferred.

In the above formula (2), X represents a group selected from the group consisting of a sulfonic acid group (-SO₃H), a sulfuric acid group (-OSO₃H), a sulfurous acid group (-OSO₂H), and salt groups thereof. From the viewpoints of acid dissociation (that is, ease of anionization) and even the solvent solubility of the copolymer, X preferably represents a group selected from the group consisting of a sulfonic acid group, a sulfuric acid group, and salt groups thereof. From the viewpoint of easy availability of monomers, X more preferably represents a sulfonic acid group or a salt group thereof. Here, the salt is not particularly limited, and for example, the salt may be an alkali metal salt (sodium salt, potassium salt, or the like) of the above group.

Examples of the compound represented by the above formula (2) include 2-(meth)acrylamide-2-methyl-1-propanesulfonic acid, 1-[(meth)acryloyloxymethyl]-1-propanesulfonic acid, 2-[(meth)acryloyloxy]-2-propanesulfonic acid, 3-[(meth)acryloyloxy]-1-methyl-1-propanesulfonic acid, 2-sulfoethyl (meth)acrylate, 3-sulfopropyl (meth)acrylate, and salts thereof (preferably a sodium salt or a potassium salt). Among these, 2-(meth)acrylamide-2-methyl-1-propanesulfonic acid or a salt thereof (particularly alkali metal salt) is preferred, and 2-acrylamide-2-methyl-1-propanesulfonic acid or a salt thereof (particularly sodium salt) is more preferred. These compounds may be used alone or in combination of two or more types thereof.

The compound represented by the above formula (2) may be either a synthetic product or a commercially available product, and the commercially available product is available from Tokyo Chemical Industry Co., Ltd., Sigma-Aldrich Co. LLC., or the like.

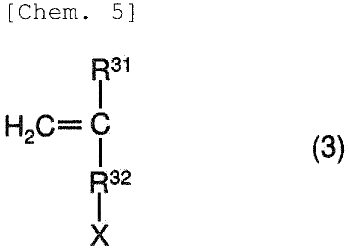

In the above formula (3), R³¹ represents a hydrogen atom or a methyl group.

In the above formula (3), R³² represents a single bond or a linear or branched alkylene group having 1 to 20 carbon atoms, preferably a single bond or a linear or branched alkylene group having 1 to 12 carbon atoms, more preferably a single bond or a linear or branched alkylene group having 1 to 8 carbon atoms, still more preferably a single bond or a linear or branched alkylene group having 1 to 4 carbon atoms, and particularly preferably a single bond. Here, since specific examples of the alkylene group are the same as those for the above formula (2), description thereof will be omitted here.

In the above formula (3), X represents a group selected from the group consisting of a sulfonic acid group (-SO₃H), a sulfuric acid group (-OSO₃H), a sulfurous acid group (-OSO₂H), and salt groups thereof. From the viewpoints of the acid dissociation (that is, the ease of anionization) and even the solvent solubility of the copolymer, X preferably represents a group selected from the group consisting of a sulfonic acid group, a sulfuric acid group, and salt groups thereof. From the viewpoint of easy availability of monomers, X more preferably represents a sulfonic acid group or a salt group thereof.

Examples of the compound represented by the above formula (3) include vinyl sulfonic acid, allyl sulfonic acid, methallyl sulfonic acid, 2-propene-1-sulfonic acid, 2-methyl-2-propene-1-sulfonic acid, and salts thereof. These compounds may be used alone or in combination of two or more types thereof.

The compound represented by the above formula (3) may be either a synthetic product or a commercially available product, and the commercially available product is available from Asahi Kasei Finechem Co., Ltd., Tokyo Chemical Industry Co., Ltd. (for example, sodium salt of 2-methyl-2-propene-1-sulfonic acid), and the like.

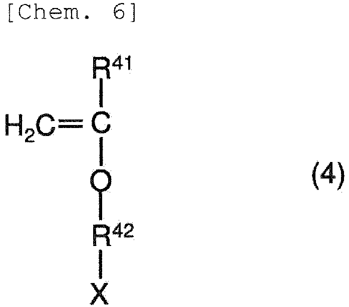

In the above formula (4), R⁴¹ represents a hydrogen atom or a methyl group.

In the above formula (4), R⁴² represents a linear or branched alkylene group having 1 to 20 carbon atoms, preferably a linear or branched alkylene group having 1 to 12 carbon atoms, more preferably a linear or branched alkylene group having 1 to 8 carbon atoms, and still more preferably a linear or branched alkylene group having 1 to 6 carbon atoms. Here, since specific examples of the alkylene group are the same as those for the above formula (2), description thereof will be omitted here.

In the above formula (4), X represents a group selected from the group consisting of a sulfonic acid group (-SO₃H), a sulfuric acid group (-OSO₃H), a sulfurous acid group (-OSO₂H), and salt groups thereof. From the viewpoints of the acid dissociation (that is, the ease of anionization) and even the solvent solubility of the copolymer, X preferably represents a group selected from the group consisting of a sulfonic acid group, a sulfuric acid group, and salt groups thereof. From the viewpoint of easy availability of monomers, X more preferably represents a sulfonic acid group or a salt group thereof.

Examples of the compound represented by the above formula (4) include 2-sulfoxyethyl vinyl ether, 3-sulfoxy-n-propyl vinyl ether, and salts thereof. These compounds may be used alone or in combination of two or more types thereof.

As the compound represented by the above formula (4), either a synthetic product or a commercially available product may be used.

In the hydrophilic copolymer, when the total of the structural units derived from all the monomers is 100 mol%, a content of the structural unit derived from the monomer B is preferably 0.1 mol% to 99 mol%, more preferably 0.2 mol% to 99 mol%, still more preferably 0.5 mol% to 99 mol%, and particularly preferably 1 mol% to 99 mol%. Within such a range, the balance between the lubricating property and the solvent solubility is good. Note that when the structural unit (B) is constituted by two or more types of structural units (B), a composition of the above structural unit (B) occupies a ratio (molar ratio (mol%)) of all the structural units (B) with respect to the total of the structural units derived from all the monomers (100 mol%). The mol% is substantially equivalent to a ratio of a charge amount (mol) of the monomer B with respect to the total charge amount (mol) of all the monomers in the production of the polymer.

### (Polymerizable Monomer (C))

The hydrophilic copolymer contains the structural unit derived from the polymerizable monomer (C) having a photoreactive group (structural unit (C)). Here, the structural unit (C) constituting the hydrophilic copolymer may be one type alone or a combination of two or more types. That is, the structural unit (C) may be constituted by only one type of structural unit (C), or may be constituted by two or more types of structural units (C). Note that a plurality of structural units (C) may be present in a block shape or in a random shape.

The polymerizable monomer (C) (monomer C) is a polymerizable monomer having a photoreactive group. Here, the "photoreactive group" refers to a group that can generate reactive species such as radicals, nitrenes, and carbenes by being irradiated with active energy rays, and react with a material constituting a layer adjacent to the surface lubricious layer (for example, the substrate layer) and the polymer containing a structural unit derived from acrylamide in the surface lubricious layer to form a chemical bond. Accordingly, the surface lubricious layer containing the hydrophilic copolymer can be firmly immobilized to an adjacent layer (for example, the substrate layer). The polymer containing a structural unit derived from acrylamide can be firmly immobilized into the surface lubricious layer. Therefore, the medical device according to the invention can exhibit sufficient durability (lubrication retaining property). The monomer C preferably has an ethylenically unsaturated group such as a (meth)acryloyl group, a vinyl group, or an allyl group, in addition to the above photoreactive group.

Examples of the photoreactive group include an azide group, a diazo group, a diazirine group, a ketone group, and a quinone group.

Examples of the azide group include an aryl azide group of phenyl azide and 4-fluoro-3-nitrophenyl azide, an acyl azide group of benzoyl azide and p-methylbenzoyl azide, an azidoformate group of ethyl azideformate and phenyl azideformate, a sulfonyl azide group of benzenesulfonyl azide, and a phosphoryl azide group of diphenylphosphoryl azide and diethyl phosphoryl azide.

Examples of the diazo group include a group derived from diazoalkanes such as diazomethane and diphenyldiazomethane, diazoketones such as diazoacetophenone and 1-trifluoromethyl-1-diazo-2-pentanone, diazoacetates such as t-butyldiazoacetate and phenyldiazoacetate, and α-diazoacetoacetates such as t-butyl-α-diazoacetoacetate.

Examples of the diazirine group include a group derived from 3-trifluoromethyl-3-phenyldiazirine.

Examples of the ketone group include a group having a structure such as acetophenone, benzophenone, anthrone, xanthine, and thioxanthone.

Examples of the quinone group include a group derived from anthraquinone.

These photoreactive groups are appropriately selected depending on the type of the substrate layer of the medical device and the like. For example, when the substrate layer is made of a polyolefin resin such as a polyethylene resin, a polyamide resin, a polyurethane resin, a polyester resin, or the like, the photoreactive group is preferably a ketone group or a phenyl azide group, and more preferably a group having a benzophenone structure (a benzophenone group) from the viewpoint of easy availability of monomers. That is, in a preferred embodiment of the invention, the polymerizable monomer (C) has a group having a benzophenone structure.

Examples of the monomer C include 2-azidoethyl (meth)acrylate, 2-azidopropyl (meth)acrylate, 3-azidopropyl (meth)acrylate, 4-azidobutyl (meth)acrylate, 4-(meth)acryloyloxybenzophenone (MBP), 4-(meth)acryloyloxyethoxybenzophenone, 4-(meth)acryloyloxy-4'-methoxybenzophenone, 4-(meth)acryloyloxyethoxy-4'-methoxybenzophenone, 4-(meth)acryloyloxy-4'-bromobenzophenone, 4-(meth)acryloyloxyethoxy-4'-bromobenzophenone, 4-styrylmethoxybenzophenone, 4-(meth)acryloyloxythioxanthone, and 2-(meth)achryloyloxyethyl-4-azidobenzoate.

As the monomer C, either a synthetic product or a commercially available product may be used, and the commercially available product is available from MCC UNITEC Co., Ltd. or the like.

In the hydrophilic copolymer, when the total of the structural units derived from all the monomers is 100 mol%, a content of the structural unit derived from the monomer C is preferably 0.1 mol% to 40 mol%, more preferably 0.1 mol% to 30 mol%, still more preferably 0.1 mol% to 25 mol%, and particularly preferably 0.1 mol% to 20 mol%. Within such a range, the hydrophilic copolymer can be sufficiently bonded to a material constituting the layer adjacent to the surface lubricious layer (in particular, the substrate layer), and thus the surface lubricious layer can be more firmly immobilized to the adjacent layer (in particular, the substrate layer). Therefore, the medical device according to the invention can exhibit sufficient durability (lubrication retaining property). Within such a range, a sufficient amount of other monomers (the monomers A and B) can be present, so that the sufficient lubricating property and durability of the monomer A and the solvent solubility of the monomer B in the hydrophilic copolymer can be more effectively improved. Note that when the structural unit (C) is constituted by two or more types of structural units (C), a composition of the above structural unit (C) occupies a ratio (molar ratio (mol%)) of all the structural units (C) with respect to the total of the structural units derived from all the monomers (100 mol%). The mol% is substantially equivalent to a ratio of a charge amount (mol) of the monomer C with respect to the total charge amount (mol) of all the monomers in the production of the polymer.

The hydrophilic copolymer may contain a structural unit derived from a polymerizable monomer other than the above monomer A, monomer B, and monomer C (hereinafter, also referred to as "other monomer") in a range that does not impair the effects of the invention. In the hydrophilic copolymer according to the invention, a content of the structural unit derived from the other monomer is preferably less than 10 mol%, more preferably less than 5 mol%, and still more preferably less than 1 mol% (lower limit: more than 0 mol%), with respect to 100 mol%, which is the total of the structural units derived from all the monomers. Note that when the structural unit derived from the other monomer is constituted by two or more types of structural units, a composition of the above structural unit derived from the other monomer occupies a ratio (molar ratio (mol%)) of all the structural units derived from the other monomer with respect to the total of the structural units derived from all the monomers (100 mol%) . Preferably, the hydrophilic copolymer according to the invention is constituted only by the monomer A, the monomer B, and the monomer C (the composition of the other monomer = 0 mol%). Note that the mol% is substantially equivalent to a ratio of a charge amount (mol) of another monomer with respect to the total charge amount (mol) of all the monomers in the production of the polymer.

A terminal end of the hydrophilic copolymer is not particularly limited and is appropriately defined depending on types of raw materials to be used, and is usually a hydrogen atom. A structure of the copolymer is not particularly limited, and may be any of a random copolymer, an alternating copolymer, a periodic copolymer, and a block copolymer.

A weight average molecular weight (Mw) of the hydrophilic copolymer is preferably several thousand to several million, more preferably 1,000 to 1,000,000, and particularly preferably 5,000 to 500,000. In the invention, the "weight average molecular weight" shall be a value measured by gel permeation chromatography (GPC) using polyethylene glycol as a standard substance.

### [Method for Manufacturing Hydrophilic Copolymer]

A method for manufacturing the hydrophilic copolymer is not particularly limited, and known polymerization methods such as radical polymerization, anionic polymerization, and cationic polymerization can be adopted. The radical polymerization that is easy in production is preferably used.

As the polymerization method, a method of copolymerizing the above monomer A, monomer B, monomer C, and if necessary, the other monomer by stirring and heating together with a polymerization initiator in a polymerization solvent is usually adopted.

A polymerization temperature is not particularly limited, and is preferably 25°C to 100°C, and more preferably 30°C to 80°C. A polymerization time is also not particularly limited, and is preferably 30 minutes to 24 hours, and more preferably 1 hour to 8 hours.

The polymerization solvent is preferably water, and an aqueous solvent such as alcohols such as methanol, ethanol, propanol, n-butanol, and 2,2,2-trifluoroethanol. From the viewpoint of dissolving raw materials to be used for the polymerization, these polymerization solvents may be used alone or in combination of two or more types thereof.

A concentration of the polymerizable monomers is not particularly limited, and a total solid content (g) of each polymerizable monomer with respect to the polymerization solvent (mL) is preferably 0.05 g/mL to 1 g/mL, and more preferably 0.1 g/mL to 0.5 g/mL. The preferred ratio of the charge amount (mol) of each monomer to the total charge amount (mol) of all the monomers is as described above.

A reaction solution containing the polymerizable monomers may be subjected to a degassing treatment before the polymerization initiator is added. The degassing treatment may be performed by, for example, bubbling the reaction solution with an inert gas such as nitrogen gas and argon gas for approximately 0.5 hours to 5 hours. During the degassing treatment, the reaction solution may be heated to approximately 30°C to 100°C.

Known polymerization initiators in the related art can be used in the production of the polymer, and the polymerization initiator is not particularly limited. For example, an azo-based polymerization initiator such as 2,2'-azobisisobutyronitrile, 2,2'-azobis(4-methoxy-2,4-dimethylvaleronitrile), 4,4'-azobis(4-cyanovaleric acid), and 2,2'-azobis(2,4-dimethylvaleronitrile), and a redox-based polymerization initiator in which a reducing agent such as sodium sulfite, sodium hydrogen sulfite, and ascorbic acid is combined with an oxidizing agent such as a persulfate such as potassium persulfate (KPS), sodium persulfate, and ammonium persulfate, and a peroxide such as hydrogen peroxide, t-butyl peroxide, and methyl ethyl ketone peroxide can be used.

A blending amount of the polymerization initiator is preferably 0.001 mol% to 10 mol%, and more preferably 0.01 mol% to 5 mol% with respect to a total amount (mol) of the polymerizable monomers.

Further, if necessary, a chain transfer agent, a polymerization rate adjusting agent, a surfactant, and other additives may be appropriately used in the polymerization.

An atmosphere in which the polymerization reaction is performed is not particularly limited, and the polymerization reaction can be performed in air atmosphere, an atmosphere of an inert gas such as nitrogen gas and argon gas, and the like. During the polymerization reaction, the reaction solution may be stirred.

The copolymer may be precipitated during the polymerization reaction. The copolymer after polymerization can be purified by a general purification method such as a reprecipitation method, a dialysis method, an ultrafiltration method, and an extraction method.

The copolymer after purification can be dried by any method such as freeze drying, vacuum drying, spray drying, and heat drying, and from the viewpoint of having a small influence on physical properties of the polymer, freeze drying or vacuum drying is preferred.

Unreacted monomers contained in the obtained copolymer are preferably 0.01 wt% or less with respect to the total amount of the copolymer. A smaller amount of unreacted monomers is preferred (lower limit: 0 wt%). A content of the remaining monomers can be measured by a known method such as high performance liquid chromatography.

### [Method for Manufacturing Medical Device]

A method for manufacturing the medical device according to the invention is not particularly limited except that the surface lubricious layer is formed using the above hydrophilic copolymer and polymer containing a structural unit derived from acrylamide, and a known method can be applied in the same manner or after appropriate modification. For example, preferred is a method in which a coating liquid is prepared by dissolving the hydrophilic copolymer and the polymer containing a structural unit derived from acrylamide in a solvent, and is coated onto the substrate layer of the medical device to form the surface lubricious layer. That is, the invention also provides the method for manufacturing the medical device according to the invention, the method including coating a coating liquid containing the polymer containing a structural unit derived from acrylamide and the hydrophilic copolymer onto the substrate layer to form the surface lubricious layer. With such a method, the lubricating property and the durability (lubrication retaining property) can be imparted to the surface of the medical device.

### (Coating Step for Surface Lubricious Layer)

Here, the coating liquid is prepared by dissolving the hydrophilic copolymer and the polymer containing a structural unit derived from acrylamide (acrylamide-based polymer) in a solvent, and is coated onto the substrate layer. In the above method, the solvent used for dissolving the hydrophilic copolymer and the acrylamide-based polymer is preferably water, a lower alcohol, or a mixed solvent of water and a lower alcohol from the viewpoints of working safety (low toxicity) and solubility. Here, the lower alcohol refers to a primary alcohol having 1 to 3 carbon atoms, that is, methanol, ethanol, n-propanol, or isopropanol. The above lower alcohols may be used alone or in combination of two or more types thereof. Here, the hydrophilic copolymer and the acrylamide-based polymer may be added to the solvent together, may be sequentially added to the same solvent (the hydrophilic copolymer and then the acrylamide-based polymer, or the acrylamide-based polymer and then the hydrophilic copolymer), or the hydrophilic copolymer and the acrylamide-based polymer may be dissolved in different solvents and then mixed with each other. Note that when the hydrophilic copolymer and the acrylamide-based polymer are used as different solvents, the solvents may be the same as or different from each other, and are preferably the same in consideration of ease of operation and the like.

A lower limit of a concentration of the hydrophilic copolymer in the coating liquid is preferably 1 wt% or more, more preferably 1.5 wt% or more, still more preferably 2.0 wt% or more, and particularly preferably 2.5 wt% or more. Within such a range, a strong and uniform chemical bond can be formed with the material constituting the substrate layer by subsequent irradiation with active energy rays (immobilizing step for surface lubricious layer). As a result, a medical device having an excellent lubricating property and excellent durability (lubrication retaining property) can be obtained. An upper limit of the concentration of the hydrophilic copolymer in the coating liquid is not particularly limited, and is preferably 30 wt% or less, more preferably 20 wt% or less, still more preferably 15 wt% or less, and particularly preferably 12.5 wt% or less. Within such a range, the coating liquid is excellent in terms of coatability and production efficiency.

A concentration of the polymer containing a structural unit derived from acrylamide in the coating liquid is not particularly limited, and is preferably 0.005 wt% to 10 wt%, and more preferably 0.5 wt% to 3 wt%. Within such a range, the coatability of the coating liquid is good, and the obtained surface lubricious layer can exhibit a sufficient water retention effect (therefore, the surface lubricious layer can exhibit an excellent lubricating property even under a high load condition).

A mixing ratio of the hydrophilic copolymer with respect to the polymer containing a structural unit derived from acrylamide in the coating liquid is not particularly limited, and is preferably the same mixing ratio as described in the section of Surface Lubricious Layer.

A coating amount of the coating liquid is not particularly limited, and is preferably an amount that corresponds to the thickness of the above surface lubricious layer.

Before coating the coating liquid, the surface of the substrate layer may be treated in advance by an ultraviolet irradiation treatment, a plasma treatment, a corona discharge treatment, a flame treatment, an oxidation treatment, a silane coupling treatment, a phosphoric acid coupling treatment, or the like. When the solvent of the coating liquid is only water, it is difficult to coat the coating liquid onto the surface of the hydrophobic substrate layer, and the surface of the substrate layer is made hydrophilic by performing a plasma treatment on the surface of the substrate layer. Accordingly, wettability of the coating liquid to the surface of the substrate layer is improved, and a uniform surface lubricious layer can be formed. By applying the above treatment to the surface of the substrate layer, which does not have any C-H bond of a metal, a fluorine resin, or the like, a covalent bond with the photoreactive group of the hydrophilic copolymer can be formed.

A method for coating the coating liquid onto the surface of the substrate layer is not particularly limited, and a known method in the related art can be applied, such as a coating printing method, an immersion method (dipping method, dip coating method), a spraying method (spray method), a spin coating method, and a mixed solution impregnated sponge coating method. Among these, an immersion method (dipping method, dip coating method) is preferred.

### (Drying Step for Surface Lubricious Layer)

As described above, it is preferable that, after immersing the substrate layer in the coating liquid, the substrate layer is taken out from the coating liquid and a coating film is dried. Drying conditions are not particularly limited as long as the solvent can be removed from the coating film, and a warm air treatment may be performed using a dryer or the like, or natural drying may be performed. A pressure condition during the drying is also not limited at all, and the drying may be performed under a normal pressure (atmospheric pressure), or under a pressure or a reduced pressure. As a drying unit (device), for example, an oven, a decompression dryer, or the like can be used, and in the case of natural drying, no drying unit (device) is particularly required.

### (Immobilizing Step for Surface Lubricious Layer)

The coating film after the above drying step is irradiated with active energy rays. Accordingly, the photoreactive group of the hydrophilic copolymer (monomer C) in the surface lubricious layer is activated, and a chemical bond is formed between the photoreactive group and a hydrocarbon group of the material constituting the substrate layer and a hydrocarbon group of the polymer containing a structural unit derived from acrylamide (acrylamide-based polymer) in the surface lubrication layer. For example, a case of a combination of the photoreactive group having a benzophenone structure of the hydrophilic copolymer and the acrylamide-based polymer in the surface lubricious layer will be described. When the hydrophilic copolymer has the photoreactive group having a benzophenone structure, two radicals are generated in the photoreactive group of the hydrophilic copolymer by the irradiation with ultraviolet rays. One of these radicals abstracts the hydrogen atom from the hydrocarbon group in the acrylamide-based polymer, and instead one radical is generated in the acrylamide-based polymer. Then, the remaining radical in the photoreactive group and the radical generated in the acrylamide-based polymer are bonded to each other, whereby a covalent bond is formed between the photoreactive group of the hydrophilic copolymer and the acrylamide-based polymer in the surface lubricious layer. With such a chemical bond between the acrylamide-based polymer and the hydrophilic copolymer in the surface lubricious layer, the acrylamide-based polymer is firmly immobilized in the surface lubricious layer. In addition, one of the two radicals of the hydrophilic copolymer generated by the irradiation with the ultraviolet rays abstracts the hydrogen atom from the hydrocarbon group in the material constituting the substrate layer, and instead one radical is generated in the material constituting the substrate layer. Then, the remaining radical in the photoreactive group of the hydrophilic copolymer and the radical generated in the material constituting the substrate layer are bonded to each other, whereby a covalent bond is formed between the photoreactive group of the hydrophilic copolymer in the surface lubricious layer and the material constituting the substrate layer. As a result, the surface lubricious layer is firmly immobilized to the substrate layer. Therefore, the surface lubricious layer can effectively exhibit the water retention effect of the acrylamide-based polymer and can exhibit an excellent lubricating property. The water retention effect of the acrylamide-based polymer can be maintained for a long period of time, and the excellent durability (lubrication retaining property) can also be exhibited.

Examples of the active energy rays include ultraviolet rays (UV), electron beams, and gamma rays, and are preferably ultraviolet rays or electron beams, and more preferably ultraviolet rays in consideration of an influence on a human body. When the active energy rays are ultraviolet rays, a wavelength at which the photoreactive group can be activated can be appropriately selected as an irradiation wavelength. Specifically, a wavelength range of the ultraviolet rays is preferably 200 nm to 400 nm, and more preferably 220 nm to 390 nm. The irradiation with ultraviolet rays is preferably performed under a temperature condition of 10°C to 100°C, and more preferably 20°C to 80°C. An irradiation intensity of the ultraviolet rays is not particularly limited, and is preferably 1 mW/cm² to 5000 mW/cm², more preferably 10 mW/cm² to 1000 mW/cm², and still more preferably 50 mW/cm² to 500 mW/cm². An integrated light amount of the ultraviolet rays is not particularly limited, and is preferably 200 mJ/cm² to 200,000 mJ/cm², and more preferably 1,000 mJ/cm² to 100,000 mJ/cm². Examples of a device for emitting the ultraviolet rays include a high-pressure mercury lamp, a low-pressure mercury lamp, a metal halide lamp, a xenon lamp, and a halogen lamp. Note that a method for emitting the active energy rays is not particularly limited, and the irradiation may be performed from one direction, or from multiple directions, or the irradiation may be performed while rotating an irradiation source, or while rotating an object to be irradiated (one in which the coating film of the surface lubricious layer is formed on the substrate layer).

After performing the above irradiation with active energy rays, the coating film may be washed with a solvent (for example, the solvent used for preparing the coating liquid) to remove the unreacted hydrophilic copolymer.

The immobilization of the coating film (surface lubricious layer) to the substrate layer can be confirmed by using a known analytical method such as FT-IR, XPS, and TOF-SIMS. For example, the immobilization can be confirmed by performing FT-IR measurement before and after the irradiation with active energy rays and comparing ratios of a peak of bonds formed by the irradiation with active energy rays with respect to a peak of invariant bonds.

With the above method, in the medical device according to the invention, the surface lubricious layer containing the hydrophilic copolymer and the polymer containing a structural unit derived from acrylamide is formed on the surface of the substrate layer.

### [Use of Medical Device]

The medical device according to the invention can be used in contact with a body fluid, blood, and the like. The surface thereof has a lubricating property in an aqueous liquid such as a body fluid or physiological saline, and can enhance operability and reduce damage to tissue mucosa. Specific examples include a catheter, a stent, and a guide wire to be used in blood vessels. That is, in one embodiment of the invention, the medical device is a catheter, a stent, or a guide wire. The medical device is also exemplified by the following.
(a) Catheters to be orally or nasally inserted or allowed to indwell in a digestive organ, such as stomach tube catheters, feeding catheters, and tubes for tube feeding.
(b) Catheters to be orally or nasally inserted or allowed to indwell in a respiratory tract or trachea, such as oxygen catheters, oxygen cannulas, tubes and cuffs of tracheal tubes, tubes and cuffs of tracheotomy tubes, and tracheal aspiration catheters.
(c) Catheters to be inserted or allowed to indwell in a urethra or ureter, such as urethra catheters, urinary catheters, and catheters and balloons of urethra balloon catheters.
(d) Catheters to be inserted or allowed to indwell in various lumens in living bodies, organs, and tissues, such as suction catheters, drain catheters, and rectum catheters.
(e) Catheters to be inserted or allowed to indwell in a blood vessel, such as indwelling needles, IVH catheters, thermodilution catheters, angiography catheters, vasodilation catheters, and dilators or introducers, or guide wires, stylets, and the like for the catheters.
(f) Artificial tracheae, artificial bronchi, and the like.
(g) Medical devices for extracorporeal circulation therapy (artificial lungs, artificial hearts, artificial kidneys, and the like) and circuits therefor.

### Examples

Hereinafter, the invention will be specifically described with reference to Examples, but the invention is not limited to these Examples. Note that parts and % in Examples are all by weight. In the following examples, unless otherwise defined, conditions for allowing to stand at room temperature are all at 23°C and 55% RH.

### Production Example 1: Production of Hydrophilic Copolymer (A)

In 10 mL of a 2,2,2-trifluoroethanol/water (9/1 v/v) mixed solvent, 1.82 g (6.5 mmol) of [2-(methacryloyloxy)ethyl]dimethyl-(3-sulfopropyl)ammonium hydroxide (MSPB) manufactured by Sigma-Aldrich Co. LLC., 1.46 g (3.2 mmol) of a 50 wt % aqueous solution of sodium 2-acrylamide-2-methyl-1-propanesulfonate (AMPS(Na)) manufactured by Sigma-Aldrich Co. LLC., and 0.080 g (0.3 mmol) of 4-methacryloyloxybenzophenone (MBP) manufactured by MCC UNITECH Co., Ltd. were dissolved to prepare a reaction solution. Next, the reaction solution was charged into a 30 mL eggplant-shaped flask, oxygen was removed by sufficient nitrogen bubbling, 2.8 mg (0.010 mmol) of a polymerization initiator 4,4'-azobis(4-cyanovaleric acid) was added, followed by immediate sealing, and polymerization was carried out in a water bath at 75°C for 3 hours. Next, the solution was subjected to reprecipitation in acetone, and the supernatant was removed by decantation to obtain a copolymer (A).

The composition of the obtained copolymer (A) was MSFB:AMPS(Na):MPB = 65:32:3 in terms of mol%. Here, the obtained copolymer (A) corresponds to the hydrophilic copolymer contained in the surface lubricious layer according to the invention. The weight average molecular weight (Mw) of the obtained copolymer (A) was measured by GPC, and was 180,000 in terms of polyethylene glycol.

### Production Example 2: Production of Hydrophilic Copolymer (B)

In 10 mL of a 2,2,2-trifluoroethanol/water (9/1 v/v) mixed solvent, 1.99 g (6.5 mmol) of a [3-(methacryloylamino)propyl]dimethyl(3-sulfobutyl)ammonium hydroxide inner salt (MSBB) manufactured by FUJIFILM Wako Pure Chemical Cooperation, 1.46 g (3.2 mmol) of a 50 wt % aqueous solution of sodium 2-acrylamide-2-methyl-1-propanesulfonate (AMPS(Na)) manufactured by Sigma-Aldrich Co. LLC., and 0.080 g (0.3 mmol) of 4-methacryloyloxybenzophenone (MBP) manufactured by MCC UNITECH Co., Ltd. were dissolved to prepare a reaction solution. Next, the reaction solution was charged into a 30 mL eggplant-shaped flask, oxygen was removed by sufficient nitrogen bubbling, 2.8 mg (0.010 mmol) of a polymerization initiator 4,4'-azobis(4-cyanovaleric acid) was added, followed by immediate sealing, and polymerization was carried out in a water bath at 75°C for 3 hours. Next, the solution was subjected to reprecipitation in acetone, and the supernatant was removed by decantation to obtain a copolymer (B).

The composition of the obtained copolymer (B) was MSBB:AMPS(Na):MPB = 65:32:3 in terms of mol%. Here, the obtained copolymer (B) corresponds to the hydrophilic copolymer contained in the surface lubricious layer according to the invention. The weight average molecular weight (Mw) of the obtained copolymer (B) was measured by GPC, and was 190,000 in terms of polyethylene glycol.

### [Example 1]

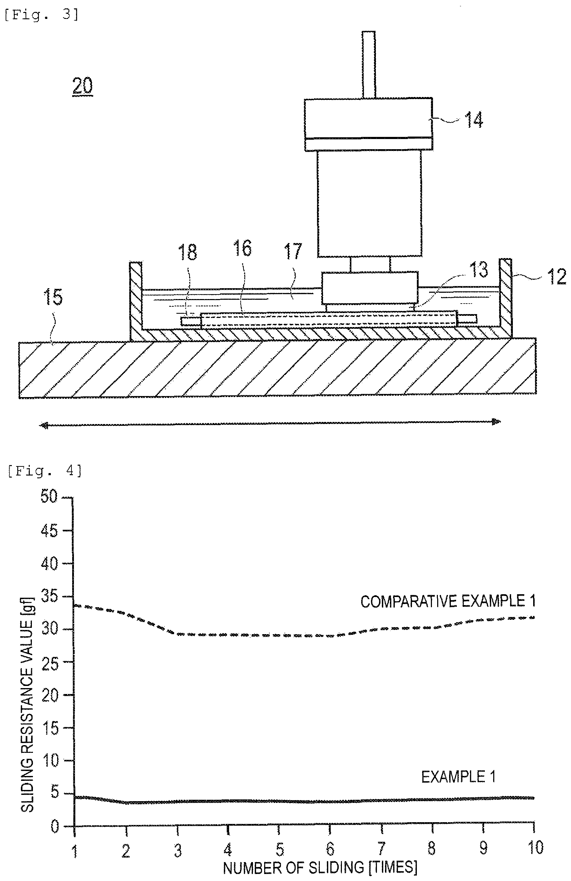

The copolymer (A) obtained in Production Example 1 (corresponding to the hydrophilic copolymer according to the invention) was dissolved in ethanol/water (3/7 w/w) so as to be 5 wt%, and polyacrylamide manufactured by Sigma-Aldrich Co. LLC. (number average molecular weight (Mn) = 150,000) was dissolved in ethanol/water (3/7 w/w) so as to be 1 wt%, to prepare a coating liquid (1). Next, a polyamide tube (having an outer diameter of 2.4 mm and a length of 70 mm) was dipped in the coating liquid (1), and was taken out at a rate of 10 mm/sec. Next, the polyamide tube was dried at room temperature for 60 seconds to remove the solvent. Next, the polyamide tube was irradiated with ultraviolet rays (UV) having a wavelength of 365 nm and an irradiation output of 105 mW/cm² under conditions of an irradiation distance of 250 mm and a sample rotation rate of 3 mm/sec for 30 minutes, so as to form a surface lubricious layer on the polyamide tube (polyamide tube (1)). A mixing ratio (weight ratio) of polyacrylamide to the hydrophilic copolymer in the surface lubricious layer of the polyamide tube (1) is 1:5. As a UV irradiation device, ECE2000 (high pressure mercury lamp) manufactured by Dymax Corporation was used. Next, the obtained sample (polyamide tube (1)) was evaluated for the lubricating property and durability (lubrication retaining property) using a friction meter (Handy Tribo Master TL201 manufactured by Trinity-Lab Inc.) 20 shown in Fig. 3 according to the following method. Results are shown in Fig. 4.

That is, a core material 18 was inserted into the above sample (polyamide tube (1)) to prepare a sample 16.

The sample 16 was laid down in a length direction and fixed in a petri dish 12, and was immersed in physiological saline 17 having a depth that the entire sample 16 was immersed in the physiological saline. The petri dish 12 was placed on a moving table 15 of the friction meter 20 shown in Fig. 3. A silicon terminal (diameter: 10 mm) 13 was brought into contact with the sample, and a load 14 of 450 g was applied on the terminal. While the moving table 15 was subjected to 10 horizontal reciprocations under a sliding distance set to 25 mm and a sliding rate set to 16.7 mm/sec, a sliding resistance value (gf) was measured. During the reciprocations from the first time to 10th time, an average of the sliding resistance values on a forward way for each reciprocation was taken and plotted on a graph as a test force to thereby evaluate a variation in sliding resistance value during the 10 repeated slides. The polyamide tube (1) was evaluated for the lubricating property (the sliding resistance value at the first reciprocation) according to the following evaluation criteria, and a result thereof was "A".

### (Evaluation Criteria)

A: The sliding resistance value at the first reciprocation is less than 40% of that of the following Comparative Example 1.
B: The sliding resistance value at the first reciprocation is 40% or more and less than 60% of that of the following Comparative Example 1.
C: The sliding resistance value at the first reciprocation is 60% or more and less than 90% of that of the following Comparative Example 1.
D: The sliding resistance value at the first reciprocation is 90% or more of that of the following Comparative Example 1.

### [Comparative Example 1]

A sample (comparative polyamide tube (1)) was prepared in the same manner as in Example 1 except that a coating liquid was prepared without blending polyacrylamide. The obtained sample (comparative polyamide tube (1)) was evaluated for the lubricating property and the durability (lubrication retaining property) in the same manner as in Example 1. Results are shown in Fig. 4.

According to Fig. 4, the polyamide tube (1) according to the invention has a low initial (the first reciprocation) sliding resistance value (excellent in lubricating property), and the sliding resistance value thereof hardly changes until the 10th reciprocation (excellent in durability (lubrication retaining property)). In contrast, the comparative polyamide tube (1) is excellent in durability (lubrication retaining property), but an initial sliding resistance value thereof is higher than that of the polyamide tube (1) according to the invention. This evaluation method is a method on assumption of a high load condition where a clearance between a catheter and an inner surface of a lumen in a living body is small. That is, the sliding resistance value is measured using the tube as a sample. The tube-shaped sample has a contact area with the terminal smaller than that of a sheet-shaped sample. Therefore, the tube-shaped sample has a force per unit area applied from the terminal (a larger load) larger than that of the sheet-shaped sample. Therefore, it is considered that the medical device according to the invention can exhibit excellent lubricating property and durability (lubrication retaining property) even under a high load condition where the clearance between the catheter and the inner surface of the lumen in the living body is small. Note that although the comparative polyamide tube (1) in Comparative Example 1 has a sliding resistance value at an initial stage (the first reciprocation) and up to the 10th reciprocation higher than that of the polyamide tube (1) according to the invention, it is considered that the comparative polyamide tube (1) also exhibits sufficient lubricating property and durability (lubrication retaining property) under normal conditions.

### [Example 2]

The copolymer (A) obtained in Production Example 1 (corresponding to the hydrophilic copolymer according to the invention) was dissolved in ethanol/water (3/7 w/w) so as to be 5 wt%, and polyacrylamide manufactured by Sigma-Aldrich Co. LLC. (number average molecular weight (Mn) = 40,000) was dissolved in ethanol/water (3/7 w/w) so as to be 1 wt%, to prepare a coating liquid (2).

A polyamide tube (2) was prepared in the same manner as in Example 1 except that the coating liquid (2) prepared as above was used instead of the coating liquid (1) in Example 1. A mixing ratio (weight ratio) of polyacrylamide to the hydrophilic copolymer in the surface lubricious layer of the polyamide tube (2) is 1:5.

Next, the obtained polyamide tube (2) was evaluated for the lubricating property (the sliding resistance value at the first reciprocation) in the same manner as in Example 1 and according to the same evaluation criteria, and a result thereof was "A".

### [Example 3]

The copolymer (A) obtained in Production Example 1 (corresponding to the hydrophilic copolymer according to the invention) was dissolved in ethanol/water (3/7 w/w) so as to be 5 wt%, and an acrylamide-acrylic acid-sodium acrylate copolymer (manufactured by Sigma-Aldrich Co. LLC., product name: [poly(acrylamide-co-acrylic acid) partial sodium salt], number average molecular weight (Mn) = 150,000, and acrylamide content = 84 mol%) was dissolved in ethanol/water (3/7 w/w) so as to be 1 wt%, to prepare a coating liquid (3).

A polyamide tube (3) was prepared in the same manner as in Example 1 except that the coating liquid (3) prepared as above was used instead of the coating liquid (1) in Example 1. A mixing ratio (weight ratio) of the acrylamide-based polymer to the hydrophilic copolymer in the surface lubricious layer of the polyamide tube (3) is 1:5.

Next, the obtained polyamide tube (3) was evaluated for the lubricating property (the sliding resistance value at the first reciprocation) in the same manner as in Example 1 and according to the same evaluation criteria, and a result thereof was "B".

### [Example 4]

The copolymer (B) obtained in Production Example 2 (corresponding to the hydrophilic copolymer according to the invention) was dissolved in ethanol/water (3/7 w/w) so as to be 5 wt%, and polyacrylamide manufactured by Sigma-Aldrich Co. LLC. (number average molecular weight (Mn) = 150,000) was dissolved in ethanol/water (3/7 w/w) so as to be 1 wt%, to prepare a coating liquid (4).

A polyamide tube (4) was prepared in the same manner as in Example 1 except that the coating liquid (4) prepared as above was used instead of the coating liquid (1) in Example 1. A mixing ratio (weight ratio) of polyacrylamide to the hydrophilic copolymer in the surface lubricious layer of the polyamide tube (4) is 1:5.

Next, the obtained polyamide tube (4) was evaluated for the lubricating property (the sliding resistance value at the first reciprocation) in the same manner as in Example 1 and according to the same evaluation criteria, and a result thereof was "A".

### [Example 5]

The copolymer (A) obtained in Production Example 1 (corresponding to the hydrophilic copolymer according to the invention) was dissolved in ethanol/water (3/7 w/w) so as to be 5 wt%, and polyacrylamide manufactured by Sigma-Aldrich Co. LLC. (weight average molecular weight (Mw) = 5,000,000 to 6,000,000) was dissolved in ethanol/water (3/7 w/w) so as to be 0.02 wt%, to prepare a coating liquid (5).

A polyamide tube (5) was prepared in the same manner as in Example 1 except that the coating liquid (5) prepared as above was used instead of the coating liquid (1) in Example 1. A mixing ratio (weight ratio) of polyacrylamide to the hydrophilic copolymer in the surface lubricious layer of the polyamide tube (5) is 1:250.

Next, the obtained polyamide tube (5) was evaluated for the lubricating property (the sliding resistance value at the first reciprocation) in the same manner as in Example 1 and according to the same evaluation criteria, and a result thereof was "B".

### [Example 6]

The copolymer (A) obtained in Production Example 1 (corresponding to the hydrophilic copolymer according to the invention) was dissolved in ethanol/water (3/7 w/w) so as to be 5 wt%, and polyacrylamide manufactured by Sigma-Aldrich Co. LLC. (weight average molecular weight (Mw) = 5,000,000 to 6,000,000) was dissolved in ethanol/water (3/7 w/w) so as to be 0.01 wt%, to prepare a coating liquid (6).

A polyamide tube (6) was prepared in the same manner as in Example 1 except that the coating liquid (6) prepared as above was used instead of the coating liquid (1) in Example 1. A mixing ratio (weight ratio) of polyacrylamide to the hydrophilic copolymer in the surface lubricious layer of the polyamide tube (6) is 1:500.

Next, the obtained polyamide tube (6) was evaluated for the lubricating property (the sliding resistance value at the first reciprocation) in the same manner as in Example 1 and according to the same evaluation criteria, and a result thereof was "C".

The results of the above Examples and Comparative Example are summarized in the following Table 1. Note that the lubricating property (the sliding resistance value at the first reciprocation) in Table 1 shows results when the number of samples is 2 (n = 2). When both samples are A, the lubricating property (the sliding resistance value at the first reciprocation) is indicated as "A", and when one sample is A and the other sample is B, the lubricating property (the sliding resistance value at the first reciprocation) is indicated as "B to A". In the following Table 1, the mixing ratio indicates a weight ratio of hydrophilic copolymer:acrylamide-based polymer in the surface lubricious layer. Columns for each hydrophilic copolymer and each acrylamide-based polymer indicate a concentration of each hydrophilic copolymer and each acrylamide-based polymer in the coating liquid, and the lubricating property means the lubricating property (the sliding resistance value at the first reciprocation).

### [Table 1]

**Table 1**

| | Surface lubricious layer | | | Lubricating property |
|---|---|---|---|---|
| | Hydrophilic copolymer (A) | Polyacrylamide Mn=150,000 | Mixing weight ratio | |
| Example 1 | 5 wt% | 1 wt% | 5:1 | A |
| Comparative Example 1 | 5 wt% | - | - | D |
| | | | | |
| | Hydrophilic copolymer (A) | Polyacrylamide Mn=40,000 | Mixing weight ratio | Lubricating property |
| Example 2 | 5 wt% | 1 wt% | 5:1 | A |
| | | | | |
| | Hydrophilic copolymer (A) | Poly(acrylamide-co-acrylic acid) partial sodium salt Mn=150,000 | Mixing weight ratio | Lubricating property |
| Example 3 | 5 wt% | 1 wt% | 5:1 | B |
| | | | | |
| | Hydrophilic copolymer (B) | Polyacrylamide Mn=150,000 | Mixing weight ratio | Lubricating property |
| Example 4 | 5 wt% | 1 wt% | 5:1 | A |
| | | | | |
| | Hydrophilic copolymer (A) | Polyacrylamide Mn=5,000,000 to 6,000,000 | Mixing weight ratio | Lubricating property |
| Example 5 | 5 wt% | 0.02 wt% | 250:1 | B |
| Example 6 | 5 wt% | 0.01 wt% | 500:1 | C |

The application is based on Japanese Patent Application No. 2019-151586, filed on August 21, 2019, the disclosure of which is referenced and incorporated as a whole.

### Reference Signs List

- 1:: substrate layer
- 1a:: substrate layer core portion
- 1b:: substrate surface layer
- 2:: surface lubricious layer
- 10:: medical device
- 12:: petri dish
- 13:: silicon terminal
- 14:: load
- 15:: moving table
- 16:: sample
- 17:: physiological saline
- 18:: core material
- 20:: friction meter

## Claims

1. A medical device, comprising:
a substrate layer; and
a surface lubricious layer formed on at least a part of the substrate layer and containing a polymer containing a structural unit derived from acrylamide, and a hydrophilic copolymer containing a structural unit derived from a polymerizable monomer (A) having a sulfobetaine structure, a structural unit derived from a polymerizable monomer (B) having at least one group selected from a group consisting of a sulfonic acid group (-SO₃H), a sulfuric acid group (-OSO₃H), a sulfurous acid group (-OSO₂H), and salt groups thereof, and a structural unit derived from a polymerizable monomer (C) having a photoreactive group.

2. The medical device according to claim 1, wherein
the polymer containing a structural unit derived from acrylamide is polyacrylamide.

3. The medical device according to claim 1 or 2, wherein
the polymerizable monomer (A) is a compound represented by the following formula (1): 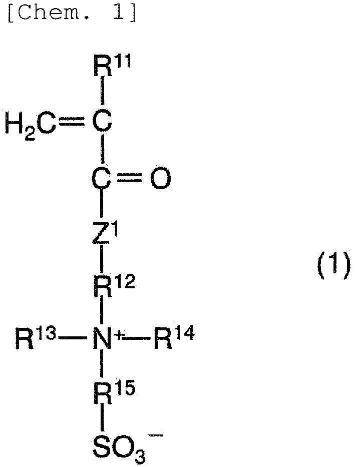 in the above formula (1),
R¹¹ represents a hydrogen atom or a methyl group,
Z¹ represents an oxygen atom or -NH-,
R¹² and R¹⁵ each independently represent a linear or branched alkylene group having 1 to 20 carbon atoms, and
R¹³ and R¹⁴ each independently represent a linear or branched alkyl group having 1 to 20 carbon atoms.

4. The medical device according to any one of claims 1 to 3, wherein
the polymerizable monomer (B) is a compound represented by the following formula (2), (3), or (4): 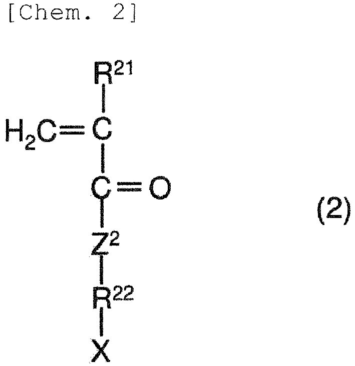 in the above formula (2),
R²¹ represents a hydrogen atom or a methyl group,
Z² represents an oxygen atom or -NH-,
R²² represents a linear or branched alkylene group having 1 to 20 carbon atoms, and
X represents a group selected from the group consisting of a sulfonic acid group (-SO₃H), a sulfuric acid group (-OSO₃H), a sulfurous acid group (-OSO₂H), and salt groups thereof, 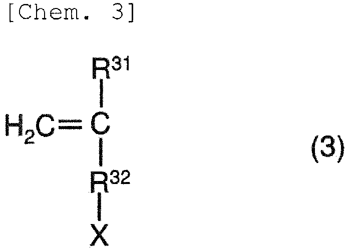 in the above formula (3),
R³¹ represents a hydrogen atom or a methyl group,
R³² represents a single bond or a linear or branched alkylene group having 1 to 20 carbon atoms, and
X represents a group selected from the group consisting of a sulfonic acid group (-SO₃H), a sulfuric acid group (-OSO₃H), a sulfurous acid group (-OSO₂H), and salt groups thereof, and 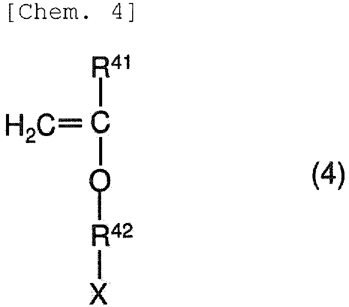 in the above formula (4),
R⁴¹ represents a hydrogen atom or a methyl group,
R⁴² represents a linear or branched alkylene group having 1 to 20 carbon atoms, and
X represents a group selected from the group consisting of a sulfonic acid group (-SO₃H), a sulfuric acid group (-OSO₃H), a sulfurous acid group (-OSO₂H), and salt groups thereof.

5. The medical device according to any one of claims 1 to 4, wherein
the polymerizable monomer (C) has a group having a benzophenone structure.

6. The medical device according to any one of claims 1 to 5, wherein
the medical device is a catheter, a stent, or a guide wire.

7. A method for manufacturing the medical device according to any one of claims 1 to 6, the method comprising:
coating a coating liquid containing the polymer containing a structural unit derived from acrylamide and the hydrophilic copolymer onto the substrate layer to form the surface lubricious layer.
